# EUROPEAN PATENT APPLICATION

(11) **EP 3 693 013 A1**
(43) Date of publication of application: **12.08.2020**
(21) Application number: 18865322.4
(22) Date of filing: 05.10.2018
(51) Int. Cl.: A61K 39/395, A61P 37/06, A61P 43/00, C07K 16/28, C07K 16/46, C12N 15/13

(54) **BISPECIFIC ANTIBODY**

(30) Priority: 06.10.2017 JP 2017196039
(71) Applicant: Ono Pharmaceutical Co., Ltd., Osaka-shi, Osaka 541-8526 (JP)
(72) Inventor: SHIBAYAMA, Shiro, Tsukuba-shi Ibaraki 300-4247 (JP); TEZUKA, Tomoya, Tsukuba-shi Ibaraki 300-4247 (JP)
(74) Representative: Grünecker Patent- und Rechtsanwälte PartG mbB
(86) International application number: PCT/JP2018/037311
(87) International publication number: WO 2019/070047

(57) **Abstract**

The present invention addresses the problem of providing a novel medical agent for prevention of auto-immune diseases, inhibition of symptom progression thereof, inhibition of recurrence thereof, or treatment thereof. As a result of conducting extensive studies, the inventors of the present invention have focused attention on PD-1/CD19 bispecific antibody as a substance with the potentiality of solving the problem, and have achieved the present invention. The PD-1/CD19 bispecific antibody according to the present invention exhibits growth-inhibitory action and immunoglobulin production inhibitory action against activated B cells, and also has inhibitory action against production of cytokine from memory T cells.

## Description

### TECHNICAL FIELD

The present invention relates to a bispecific antibody capable of specifically binding to each of PD-1 and CD19 expressed on single B cells (hereinafter, abbreviated as PD-1/CD19 bispecific antibody), and its use in pharmaceutical treatment.

### BACKGROUND ART

PD-1 is an immunosuppressive receptor belonging to an immunoglobulin family and is a molecule having a function of suppressing immune activation signals of T-cells activated by stimulation through antigen receptor. For example, analysis of PD-1 knock-out mice demonstrates that a PD-1 signal plays an important role in suppression of autoimmune diseases such as autoimmune dilated cardiomyopathy, lupus-like syndrome, autoimmune encephalomyelitis, systemic lupus erythematosus, graft-versus-host disease, type I diabetes mellitus and rheumatoid arthritis. Accordingly, it is pointed out that a substance enhancing PD-1 signal can be a prophylactic or therapeutic agent for autoimmune diseases.

On the other hand, CD19 is a membrane protein expressed on B cells and is a molecule that transmits stimuli to B cells together with a B cell receptor complex.

Bispecific antibodies recognizing PD-1 have been recognized as a substance for enhancing a PD-1 signal (Patent Literatures 1 to 3). These bispecific antibodies are composed of an antigen-recognition site of an antibody for recognizing CD3, which is a member of a T-cell receptor complex, and an antigen-recognition site of an antibody recognizing PD-1 linked to each other using genetic engineering. Bispecific antibodies enhance an inhibitory signal of PD-1 against the T-cell receptor complex by increasing the frequency of bringing PD-1 to the periphery of the T-cell receptor complex. Patent Literatures 1 to 3 also state that PD-1 bispecific antibodies can be used for prophylaxis or therapy of autoimmune diseases.

### CITATIONS LIST

Patent Literature

Patent Literature 1: International Publication No. WO2003/011911
Patent Literature 2: International Publication No. WO2004/072286
Patent Literature 3: International Publication No. WO2013/022091

### SUMMARY OF INVENTION

### TECHNICAL PROBLEMS

It is an object of the present invention to provide a new agent for prophylaxis, symptom progress-suppression, recurrence-suppression or therapy of autoimmune diseases.

### SOLUTIONS TO PROBLEMS

As a result of intensive studies, the inventors of the present invention have focused on a PD-1/CD19 bispecific antibody as a substance capable of solving such problems, thereby completing the present invention.

In other words, the present invention is as follows.
[1] A PD-1/CD19 bispecific antibody or antibody fragment thereof, containing the first arm capable of specifically binding to PD-1 (hereinafter, also abbreviated as the first arm) and the second arm capable of specifically binding to CD 19 (hereinafter, also abbreviated as the second arm).
[2] The PD-1/CD19 bispecific antibody or antibody fragment thereof according to the preceding item [1], wherein the first arm contains a heavy chain variable region (hereinafter, abbreviated as VH) and a light chain variable region (hereinafter, abbreviated as VL) of an antibody capable of specifically binding to PD-1 (hereinafter, referred to as anti-PD-1 antibody).
[3] The PD-1/CD19 bispecific antibody or antibody fragment thereof according to the preceding item [1] or [2], wherein the second arm contains a VH and VL of an antibody capable of specifically binding to CD 19 (hereinafter, referred to as anti-CD 19 antibody).
[4] The PD-1/CD19 bispecific antibody or antibody fragment thereof according to the preceding item [2] or [3], having a structure in which the VH of anti-PD-1 antibody, the VL of anti-CD 19 antibody, the VH of anti-CD 19 antibody and the VL of anti-PD-1 antibody are linked via peptide linkers or directly to each other in this order from its N-terminal.
[5] The PD-1/CD19 bispecific antibody or antibody fragment thereof according to the preceding item [2] or [3], having a structure in which the VL of anti-PD-1 antibody, the VH of anti-PD-1 antibody, the VH of anti-CD19 antibody and the VL of anti-CD19 antibody are linked via peptide linkers or directly to each other in this order from its N-terminal.
[6] The PD-1/CD19 bispecific antibody or antibody fragment thereof according to the preceding item [2] or [3], having a structure in which the VL of anti-CD19 antibody, the VH of anti-PD-1 antibody, the VL of anti-PD-1 antibody and the VH of anti-CD 19 antibody are linked via peptide linkers or directly to each other in this order from its N-terminal.
[7] The PD-1/CD19 bispecific antibody or antibody fragment thereof according to any one of the preceding items [4] to [6], having a structure in which the N-terminal of a polypeptide having a human IgG constant region from a hinge site to a CH3 region (hereinafter, also abbreviated as "IgG constant region (hinge/CH3)") is further linked via a peptide linker or directly to the C-terminal of the VL of anti-PD-1 antibody of the preceding item [4], that of the VL of anti-CD 19 antibody of the preceding item [5] or that of the VH of CD19 antibody of the preceding item [6].
[8] The PD-1/CD19 bispecific antibody or antibody fragment thereof according to the preceding item [2] or [3], having a structure in which the VL of anti-CD 19 antibody, the VH of anti-CD 19 antibody, IgG constant region (hinge/CH3), the VH of anti-PD-1 antibody and the VL of anti-PD-1 antibody are linked via peptide linkers or directly to each other in this order from its N-terminal.
[9] The PD-1/CD19 bispecific antibody or antibody fragment thereof according to the preceding item [7] or [8], further having a structure including a polypeptide having IgG constant region (hinge/CH3).
[10] The PD-1/CD19 bispecific antibody or antibody fragment thereof according to any one of the preceding items [4] to [9], wherein the peptide linker is Gly-Gly-Gly-Gly-Ser (SEQ ID NO: 1; hereinafter, abbreviated as (Gly)×4-Ser) or Gly-Gly-Gly-Gly-Ser-Gly-Gly-Gly-Gly-Ser-Gly-Gly-Gly-Gly-Ser (SEQ ID NO: 2; hereinafter, abbreviated as ((Gly)×4-Ser)×3).
[11]The PD-1/CD19 bispecific antibody or antibody fragment thereof according to any one of the preceding items [1] to [3], being in a form of diabody, bispecific sc(Fv)₂, bispecific minibody, bispecific F(ab')₂, bispecific hybrid antibody, covalent diabody (bispecific DART), bispecific (FvCys)₂, bispecific F(ab'-zipper)₂, bispecific (Fv-zipper)₂, bispecific triple-chain antibody or bispecific mAb².
[12] The PD-1/CD19 bispecific antibody or antibody fragment thereof according to any one of the preceding items [1] to [3], being in a form of bispecific hybrid antibody.
[13] The PD-1/CD19 bispecific antibody or antibody fragment thereof according to any one of the preceding items [1] to [6] and [10] to [12], being an IgG antibody.
[14] The PD-1/CD19 bispecific antibody or antibody fragment thereof according to the preceding item [13], wherein the IgG antibody is an IgG₁ antibody or IgG₄ antibody.
[15] The PD-1/CD19 bispecific antibody or antibody fragment thereof according to the preceding item [14], wherein the IgG₁ antibody is an antibody having no ADCC and/or CDC effect.
[16] The PD-1/CD19 bispecific antibody or antibody fragment thereof according to any one of the preceding items [7] to [9], wherein the isotype of the IgG constant region (hinge/CH3) is IgG₁.
[17] The PD-1/CD19 bispecific antibody or antibody fragment thereof according to any one of the preceding items [1] to [16], wherein PD-1 and CD19 are human PD-1 and human CD19, respectively.
[18] The PD-1/CD19 bispecific antibody or antibody fragment thereof according to any one of the preceding items [1] to [17], being a monoclonal antibody.
[19] The PD-1/CD19 bispecific antibody or antibody fragment thereof according to any one of the preceding items [1] to [18], being a humanized or fully human-type antibody.
[20] The PD-1/CD19 bispecific antibody or antibody fragment thereof according to any one of the preceding items [1] to [19], being an isolated antibody.
[21] The PD-1/CD19 bispecific antibody or antibody fragment thereof according to any one of the preceding items [1] to [20], wherein the first arm allows interaction between PD-1 and PD-L1, interaction with PD-1 and PD-L2, or both of the interactions.
[22] The PD-1/CD19 bispecific antibody or antibody fragment thereof according to any one of the preceding items [1] to [21], binding to PD-1 and CD19 expressed on B cells.
[23] A humanized or fully human-type isolated PD-1/CD19 bispecific antibody having the first arm capable of specifically binding to PD-1 and the second arm capable of specifically binding to CD 19,
   (a) wherein the first arm contains a VH and VL of anti-human PD-1 antibody, and the second arm contains a VH and VL of anti-human CD19 antibody,
   (b) which binds to each of PD-1 and CD19 expressed on B cells,
   (c) wherein the first arm allows interaction between PD-1 and PD-L1, and
   (d) which is in a form of bispecific hybrid antibody and/or IgG₁ or IgG₄ antibody.
[1-1] A pharmaceutical composition containing the PD-1/CD19 bispecific antibody or antibody fragment thereof selected from any one of the preceding items [1] to [22], or the humanized or fully human-type isolated PD-1/CD19 bispecific antibody of the preceding item [23], and a pharmaceutically acceptable carrier.
[2-1] A prophylactic, symptom progress-suppressive, recurrence-suppressive and/or therapeutic agent for autoimmune disease, containing the PD-1/CD19 bispecific antibody or antibody fragment thereof selected from any one of the preceding items [1] to [22], or the humanized or fully human-type isolated PD-1/CD19 bispecific antibody of the preceding item [23], as an active ingredient.
[2-2] The prophylactic, symptom progress-suppressive, recurrence-suppressive and/or therapeutic agent according to the preceding item [2-1], wherein autoimmune disease is Behcet disease, systemic lupus erythematosus, chronic discoid lupus erythematosus, multiple sclerosis (systemic scleroderma and progressive systemic scleroderma), scleroderma, polymyositis, dermatomyositis, periarteritis nodosa (polyarteritis nodosa and microscopic polyangiitis), aortitis syndrome (Takayasu arteritis), malignant rheumatoid arthritis, rheumatoid arthritis, juvenile idiopathic arthritis, spondylarthritis, mixed connective tissue disease, Sjogren syndrome, adult-onset Still's disease, vasculitis, allergic granulomatous angiitis, hypersensitivity angiitis, rheumatoid vasculitis, large-vessel vasculitis, ANCA-associated vasculitis (e.g., granulomatosis with polyangiitis and eosinophilic granulomatosis with polyangiitis), Cogan's syndrome, RS3PE syndrome, temporal arteritis, giant-cell arteritis, polymyalgia rheumatica, fibromyalgia syndrome, antiphospholipid antibody syndrome, eosinophilic fasciitis, IgG4-related diseases (e.g., primary sclerosing cholangitis and autoimmune pancreatitis), Guillain-Barre syndrome, myasthenia gravis, chronic atrophic gastritis, autoimmune hepatitis, non-alcoholic steatohepatitis, primary biliary cirrhosis, aortitis syndrome, Goodpasture's syndrome, rapidly progressive glomerulonephritis, anti-glomerular basement membrane nephritis, megaloblastic anemia, autoimmune hemolytic anemia, pernicious anemia, autoimmune neutropenia, idiopathic thrombocytopenic purpura, hyperthyroidism (Graves' disease (Basedow's disease)), Hashimoto's thyroiditis, autoimmune adrenal insufficiency, primary hypothyroidism, Addison's disease, idiopathic Addison's disease (chronic adrenal insufficiency), type I diabetes mellitus, slowly progressive type I diabetes mellitus, localized scleroderma, psoriasis, psoriatic arthritis, bullous pemphigoid, pemphigus, pemphigoid, herpes gestationis, linear IgA bullous skin disease, epidermolysis bullosa acquisita, alopecia areata, vitiligo, vitiligo vulgaris, Harada disease, autoimmune optic neuropathy, idiopathic azoospermia, recurrent fetal loss, inflammatory bowel diseases (e.g., ulcerative colitis and Crohn's disease), celiac disease, atopic dermatitis, neuromyelitis optica, chronic inflammatory demyelinating polyneuropathy, multifocal motor neuropathy, pulmonary alveolar proteinosis, autoimmune hemorrhagic disease XIII, relapsing polychondritis, sarcoidosis, ankylosing spondylitis, severe asthma, chronic urticaria, transplantation immunity, familial Mediterranean fever, eosinophilic sinusitis, dilated cardiomyopathy, food allergy, systemic mastocytosis, amyotrophic lateral sclerosis or inclusion body myositis.
[2-3] The prophylactic, symptom progress-suppressive, recurrence-suppressive and/or therapeutic agent according to the preceding item [2-1] or [2-2], being administered together with one or more agent(s) selected from steroid drugs (e.g., cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone sodium phosphate, dexamethasone palmitate, paramethasone acetate, betamethasone or the like), interferon β-1a, interferon β-1b, glatiramer acetate, mitoxantrone, azathioprine, cyclophosphamide, cyclosporin, methotrexate, cladribine, adrenocorticotropic hormone (ACTH), corticotropin, mizoribine, tacrolimus, fingolimod, alemtuzumab, immunosuppressive agents (e.g., cyclosporin, tacrolimus, fingolimod or the like), anti-rheumatic drugs (e.g., methotrexate, sulfasalazine, bucillamine, leflunomide, mizoribine and tacrolimus), and anti-cytokine drugs (e.g., infliximab, adalimumab, tocilizumab, etanercept, abatacept, golimumab and certolizumab).
[3-1] A prophylactic, symptom progress-suppressive, recurrence-suppressive and/or therapeutic agent for graft-versus-host disease (GVHD), containing the PD-1/CD19 bispecific antibody or antibody fragment thereof selected from any one of the preceding items [1] to [22], or the humanized or fully human-type isolated PD-1/CD19 bispecific antibody of the preceding item [23], as an active ingredient.
[4-1] A prophylactic, symptom progress-suppressive, recurrence-suppressive and/or therapeutic agent for autoreactive B cell mediated disease, containing the PD-1/CD19 bispecific antibody or antibody fragment thereof selected from any one of the preceding items [1] to [22], or the humanized or fully human-type isolated PD-1/CD19 bispecific antibody of the preceding item [23], as an active ingredient.
[4-2] The prophylactic, symptom progress-suppressive, recurrence-suppressive and/or therapeutic agent according to the preceding item [4-1], wherein autoreactive B cell mediated disease is systemic lupus erythematosus, Graves' disease, myasthenia gravis, autoimmune hemolytic anemia, autoimmune thrombocytopenia, asthma, cryoglobulinemia, primary bile duct sclerosis or pernicious anemia.
[5-1] An autoreactive B cell suppressive agent containing the PD-1/CD19 bispecific antibody or antibody fragment thereof selected from any one of the preceding items [1] to [22], or the humanized or fully human-type isolated PD-1/CD19 bispecific antibody of the preceding item [23], as an active ingredient.
[5-2] The autoreactive B cell suppressive agent according to the preceding item [5-1], wherein the autoreactive B cell suppression is suppression of immunoglobulin production.
[5-3] The autoreactive B cell suppressive agent according to the preceding item [5-2], wherein the immunoglobulin is IgG or IgM.
[6-1] A memory T cell activation suppressive agent containing the PD-1/CD19 bispecific antibody or antibody fragment thereof selected from any one of the preceding item [1] to [22], or the humanized or fully human-type isolated PD-1/CD19 bispecific antibody of the preceding item [23], as an active ingredient.
[6-2] The memory T cell activation suppressive agent according to the preceding item [6-1], wherein the memory T cell activation suppression is suppression of cytokine production.
[7-1] A method for prophylaxis, symptom progress-suppression, recurrence-suppression and/or therapy of autoimmune disease (particularly, autoreactive B cell mediated disease) or graft-versus-host disease (GVHD), including administering to a patient an effective amount of the PD-1/CD19 bispecific antibody or antibody fragment thereof selected from any one of the preceding items [1] to [22], or the humanized or fully human-type isolated PD-1/CD19 bispecific antibody of the preceding item [23].
[7-2] A method for suppressing autoreactive B cell or memory T cell activation, including administering to a patient an effective amount of the PD-1/CD19 bispecific antibody or antibody fragment thereof selected from any one of the preceding items [1] to [22], or the humanized or fully human-type isolated PD-1/CD19 bispecific antibody of the preceding item [23].
[8-1] The PD-1/CD19 bispecific antibody or antibody fragment thereof selected from any one of the preceding item [1] to [22], or the humanized or fully human-type isolated PD-1/CD19 bispecific antibody of the preceding item [23] for prophylaxis, symptom progress-suppression, recurrence-suppression and/or therapy of autoimmune disease (particularly, autoreactive B cell mediated disease) or graft-versus-host disease (GVHD).
[8-2] The PD-1/CD19 bispecific antibody or antibody fragment thereof selected from any one of the preceding items [1] to [22], or the humanized or fully human-type isolated PD-1/CD19 bispecific antibody of the preceding item [23] for suppression of autoreactive B cell or memory T cell activation.
[9-1] A use of the PD-1/CD19 bispecific antibody or antibody fragment thereof selected from any one of the preceding items [1] to [22], or the humanized or fully human-type isolated PD-1/CD19 bispecific antibody of the preceding item [23] in the manufacture of a prophylactic, symptom progress-suppressive, recurrence-suppressive and/or therapeutic agent for autoimmune disease (particularly, autoreactive B cell mediated disease) or graft-versus-host disease (GVHD).
[9-2] A use of the PD-1/CD19 bispecific antibody or antibody fragment thereof selected from any one of the preceding items [1] to [22], or the humanized or fully human-type isolated PD-1/CD19 bispecific antibody of the preceding item [23] in the manufacture of an autoreactive B cell suppressive agent or memory T cell activation suppressive agent.

### ADVANTAGEOUS EFFECTS OF INVENTION

The PD-1/CD19 bispecific antibody of the present invention has a PD-1 agonist effect, and also exhibits a suppressive effect on activated B cells, so that it can be used for prophylaxis, symptom progress-suppression, recurrence-suppression and/or therapy of autoimmune disease or GVHD.

### BRIEF DESCRIPTION OF DRAWINGS

Figure 1 shows a structure of B cell-targeting PD-1 agonist (B cell-targeting PD-1 agonist X and B cell-targeting PD-1 agonist M) described in Example 1. In the figure, the "VH CD19" and "VL CD19" represent a VH and VL of anti-CD19 antibody, respectively, and the "VH PD-1" and "VL PD-1" represent a VH and VL of anti-PD-1 antibody, respectively. The "L1", "L2" and "L3" represent a peptide linker linking the respective VHs and VLs, and the L1 and L3 have a structure represented by (Gly)×4-Ser and the L2 has a structure represented by ((Gly)×4-Ser)×3.
Figure 2 shows a structure of B cell-targeting PD-1 agonist M-Fc described in Example 2. In the figure, the "IgG₁(Hinge-CH3)" represents a human IgG₁ constant region having a hinge site to a CH3 region. The "L4" represents a peptide having a structure represented by (Gly)×4-Ser or ((Gly)×4-Ser)×3.Other symbols have the same meaning as shown in Figure 1.
Figure 3 shows flow cytometry showing specific binding properties of B cell-targeting PD-1 agonist X to human PD-1 and human CD19.
Figure 4 shows flow cytometry showing specific binding properties of B cell-targeting PD-1 agonist M to human PD-1 and human CD 19.
Figure 5 shows flow cytometry showing specific binding properties of B cell-targeting PD-1 agonist M-Fc to human PD-1 and human CD19.
Figure 6 shows the effect of each B cell-targeting PD-1 agonist on cell proliferation of activated human B cells. (A) B cell-targeting PD-1 agonist M (in the figure, represented by "Agonist M"), (B) B cell-targeting PD-1 agonist X (in the figure, represented by "Agonist X") and (C) B cell-targeting PD-1 agonist M-Fc (in the figure, represented by "Agonist M-Fc").
Figure 7 shows the effect of B cell-targeting PD-1 agonist M-Fc on IgM and IgG production in human B cells.
Figure 8 shows the effect of B cell-targeting PD-1 agonist M-Fc in a xenograft GVHD mouse model.
Figure 9 shows the effect of B cell-targeting PD-1 agonist on IL-2 production from human memory T cells.
Figure 10 shows the effect of B cell-targeting PD-1 agonist M-Fc on immune response to thymus-dependent antigens. The vertical axis of the graph represents the antibody titer against KLH in serum.
Figure 11 shows a structure of mouse B cell-targeting PD-1 agonist-Fc-1 described in Example 9. In the figure, the "VH mCD19" and "VL mCD19" represent a VH and VL of anti-mouse CD19 antibody, respectively, and the "VH mPD-1" and "VL mPD-1" represent a VH and VL of anti-mouse PD-1 antibody, respectively. Other symbols have the same meaning as described above.
Figure 12 shows a structure of mouse B cell-targeting PD-1 agonist-Fc-2 described in Example 9. Symbols in the figure have the same meaning as described above.
Figure 13 shows flow cytometry showing specific binding properties of a mouse B cell-targeting PD-1 agonist-Fc-1 to mouse PD-1 and mouse CD19.
Figure 14 shows flow cytometry showing specific binding properties of mouse B cell-targeting PD-1 agonist-Fc-2 to mouse PD-1 and mouse CD19.
Figure 15 represents the *in vivo* effect of each of mouse B cell-targeting PD-1 agonists-Fc-1 and -2 on immune response in NP-OVA immunized mice. The vertical axis of the graph represents the number of class switch memory B cells, "Mouse Agonist-Fc-1" represents mouse B cell-targeting PD-1 agonist-Fc-1, and "Mouse Agonist-Fc-2" represents mouse B cell-targeting PD-1 agonist-Fc-2.

### DESCRIPTION OF EMBODIMENTS

PD-1 (Programmed Cell Death-1) is a membrane protein composed of the amino acid sequence represented by GenBank accession number NP_005009 in humans. When described herein as "PD-1", unless otherwise specified, PD-1 may be used as those also including all its isoforms and those variants in which an epitope of the "first arm specifically binding to PD-1" in accordance with the present invention is conserved. In the present invention, PD-1 is preferably human PD-1.

CD 19 is a membrane protein having the amino acid sequence represented by Genbank accession number NP001761 and being expressed on B cells to form a complex with the B cell receptor in humans. When described herein as "CD19", unless otherwise specified, CD19 may be used as those also including all its isoforms and those variants in which an epitope of the "second arm specifically binding to CD 19" in accordance with the present invention is conserved. In the present invention, CD19 is preferably human CD 19.

As used herein, the "isolation" means that a substance becomes a substantially single pure component by identifying, separating and/or purifying the same from a mixture extracted from host cells, containing a plurality of or myriad components.

As used herein, the "monoclonal antibody" means an antibody obtained from a substantially homogeneous antibody population, binding to a single specific antigen.

As used herein, the "bispecific antibody" means an antibody having binding specificities to two different antigen molecules or epitopes in one molecule. The present invention relates to a bispecific antibody and antibody fragment thereof capable of specifically binding to PD-1 and CD19 expressed on B cells, respectively. In one embodiment, the present invention relates to a bispecific antibody and antibody fragment thereof capable of simultaneously specifically binding to PD-1 and CD19 expressed on B cells, respectively. In one embodiment, the present invention also relates to a bispecific antibody and antibody fragment thereof capable of specifically binding to PD-1 and CD19 expressed on single B cell, respectively.

Examples of the form of bispecific antibody include diabody, bispecific sc(Fv)₂, bispecific minibody, bispecific F(ab')₂, bispecific hybrid antibody, covalent diabody (bispecific DART), bispecific (FvCys)₂, bispecific F(ab'-zipper)₂, bispecific (Fv-zipper)₂, bispecific triple-chain antibody and bispecific mAb².

The diabody is a dimer of single-chain peptides in which VHs and VLs recognizing different antigens are linked to each other through a peptide linker (see Proc. Natl. Acad. Sci. USA (1993), Vol. 90, No.14: p.6444-6448).

The bispecific sc(Fv)₂ is a low-molecular antibody modified such that two pairs of VHNL of two antibodies recognizing different antigens are produced in a single chain form consecutive through peptide linkers (see J. Biological Chemistry (1994), Vol. 269: p.199-206).

The bispecific F(ab')₂ is a low-molecular antibody in which Fab' fragments of antibodies recognizing two different antigens are covalently bonded through, for example, disulfide bond.

The bispecific minibody is a low-molecular antibody in which low-molecular antibody fragments modified such that the constant region CH3 domains of antibodies are linked to scFvs recognizing different antigen, respectively, are covalently bound through, for example, disulfide bond on the CH3 domains (see Biochemistry (1992), Vo. 31, No. 6, p.1579-1584).

The bispecific hybrid antibody is an intact antibody in which the heavy chain/light chain complexes consisting of antibodies recognizing two different antigens, respectively, are covalently bound to each other through, for example, disulfide bond.

In the present invention, as a preferable bispecific antibody form, one having wide movable area between the two variable areas is preferable. For example, a bispecific hybrid antibody is included.

The bispecific hybrid antibody can be produced, for example, from a hybridoma produced by the hybrid hybridoma method (see US4474893). In addition, the bispecific hybrid antibody can be produced by allowing mammalian cells to co-express and secrete a total of four types of cDNAs individually encoding heavy and light chains of antibodies recognizing different antigens.

The monoclonal antibody for use in the present invention can be produced by the hybridoma method (see e.g., Kohler and Milstein et al., Nature (1975), Vol. 256, p.495-97, Hongo et al., Hybridoma (1995), Vol. 14, No. 3, p.253-260, Harlow et al., Antibodies: A Laboratory Manual, (Cold Spring Harbor Laboratory Press (1988), Vol. 2) and Hammerling et al., Monoclonal Antibodies and T-Cell Hybridomas, p.563-681 (see, Elsevier, N.Y., 1981), recombinant DNA method (see, e.g., US4816567) or phage display method (see e.g., US5223409, US5403484 and US5571698 to Ladner et al., US5427908 and US5580717 to Dower et al., US5969108 and US6172197 to McCafferty et al., and US5885793, US6521404, US6544731, US6555313, US6582915 and US6593081 to Griffiths et al.).

When administered to humans, an antibody or monoclonal antibody can be produced in the form of chimeric antibody, humanized antibody or fully human-type antibody in order to reduce or eliminate its antigenicity.

The "chimeric antibody" means an antibody in which the variable region sequence and constant region sequence are derived from different mammals, for example, an antibody in which the variable region sequence is derived from a mouse antibody and the constant region sequence is derived from a human antibody. The chimeric antibody can be produced by ligating a gene encoding an antibody variable region isolated by a publicly known technique from an antibody-producing hybridoma isolated by the above-mentioned hybridoma method, recombinant DNA method or phage display method to a gene encoding a human-derived antibody constant region using a publicly known method (see, e.g., US4816567 to Cabilly et al.).

The "humanized antibody" means an antibody in which a complementarity determining region (CDR) sequences derived from a germline type of another mammalian such as mouse were grafted onto a human framework sequence. Also in this case, the humanized antibody can be produced by ligating a gene encoding the antibody CDR regions isolated by a publicly known technique from an antibody-producing hybridoma isolated by the above-mentioned method to a gene encoding a human-derived antibody framework region using a publicly known method (see, e.g., US5225539 and US5530101 to Winter, and US5585089 and US6180370 to Queen et al.).

The "human antibody" or "fully human-type antibody" means an antibody having a variable region composed of framework regions and CDR regions, and a constant region, both derived from a human germline immunoglobulin sequence. The human antibody for use in the present invention can be produced by a method using a mouse transformed to produce a human antibody, such as Humab mice (see, e.g., US5545806, US5569825, US5625126, US5633425, US5789650, US5877397, US5661016, US5814318, US5874299 and US5770429 to Lonberg and Kay et al.), KM mouse (see, e.g., WO2002/43478 by Ishida et al.), Xeno mouse (see, e.g., US5939598, US6075181, US6114598, US6150584 and US6162963) or Tc mouse (see, e.g., Tomizuka et al., Proc. Natl. Acad. Sci. USA (2000), p.722-727). The human antibody can also be prepared using SCID mice (see, e.g., US5476996 and US5698767 to Wilson et al.) in which human immune cells are reconstituted to cause a human antibody response upon immunization. Furthermore, the human antibody for use in the present invention can also be produced by the above-mentioned phage display method.

As used herein, the "antibody fragment" of a PD-1/CD19 bispecific antibody is a part of a full-length antibody, and is an antibody having an antigen-binding portion to PD-1 and an antigen-binding portion to CD 19. For example, F(ab')₂ is included. Here, the antigen-binding portion means a minimum unit that allows the antibody to bind to its antigen, for example, which is composed of three CDRs on each of the VH and VL and framework regions arranging the CDRs such that they can recognize a target antigen based on their combination.

As used herein, the "isotype" is used to refer to an antibody class (e.g., IgM or IgG) encoded by a heavy chain constant region gene. A preferred isotype as the bispecific antibody of the present invention is IgG₁ or IgG₄. Here, preferable IgG₁ includes one from which the binding to the Fc receptor is eliminated or attenuated. Specifically, an IgG₁ antibody from which the binding to the Fc receptor is eliminated or attenuated can be obtained by substituting, deleting or inserting arbitrary amino acid in or into the heavy chain constant region. Examples of the IgG₁ antibody include one in which leucine at position 234 according to the EU numbering system is substituted with alanine and/or leucine at position 235 is substituted with alanine on each of the two heavy chain constant regions or a hinge region. Examples of such an antibody may also include one in which leucine at position 235 according to the EU numbering system is substituted with glycine and/or glycine at position 236 is substituted with arginine on each of the two heavy chain constant regions or a hinge region of the bispecific antibody. In addition, in order to reduce the heterogeneity of antibody, it is preferably one lacking its C-terminal amino acid, for example, lysine at position 447 according to the EU numbering system.

Arbitrary amino acid(s) in the Fc region of the bispecific antibody of the present invention may be substituted such that its two different heavy chains are easy to associate. Examples of the PD-1/CD19 bispecific antibody include one in which leucine at position 351 according to the EU numbering system is substituted with lysine and the 366th threonine is substituted with lysine in the constant region on the heavy chain having the first arm VH that specifically binds to PD-1, and leucine at position 351 is substituted with aspartic acid and leucine at position 368 is substituted with glutamic acid in the constant region on the heavy chain having the second arm VH that specifically binds to CD19. In addition, examples of the PD-1/CD19 bispecific antibody also include one in which leucine at position 351 according to the EU numbering system is substituted with aspartic acid and leucine at position 368 is substituted with glutamic acid in the constant region on the heavy chain having the first arm VH that specifically binds to PD-1, and leucine at position 351 is substituted with lysine and threonine at position 366 is substituted with lysine in the constant region on the heavy chain having the second arm VH that specifically binds to CD 19.

Furthermore, when the bispecific antibody of the present invention is IgG₄, a variant is more preferable in which arbitrary amino acid(s) is/are substituted, deleted or inserted in or into the heavy chain constant region so as to suppress swapping in the antibody molecule. For example, the antibody in which serine at position 226 according to the EU numbering system, located in the hinge region, is substituted with proline is preferable. In this specification, the amino acid position assigned to the CDR and framework in the variable region of the antibody is defined in accordance with Kabat (see Sequences of Proteins of Immunological Interest (National Institute of Health, Bethesda, Md., (1987) and (1991)). In addition, the amino acids in the constant region are represented in accordance with the EU numbering system (see Sequences of proteins of immunological interest, NIH Publication No. 91-3242) based on the Kabat amino acid position.

As used herein, "autoreactive B cell" refers to the B cells which produce antibodies against self-antigens.

### First arm specifically binding to PD-1

In the present specification, the "first arm capable of specifically binding to PD-1" means an antibody portion, at least including a VH and VL of anti-PD-1, capable of specifically binding to PD-1 through associating with each other, regardless that it is included as a part of an antibody or antibody fragment thereof, or exists as not such a part but a single substance itself. Examples of the first arm also include the Fab portion of an anti-PD-1 antibody. Here, the "specifically binding to PD-1" is used as a feature that an antibody can directly bind to PD-1 with the binding activity having at least an affinity (dissociation constant (Kd value)) higher than 1 × 10⁻⁵M, more preferably 1 × 10⁻⁷M, more preferably 1 × 10⁻⁹M, but does not substantially bind to at least other receptor members belonging to the so-called CD28 receptor family, such as CD28, CTLA-4 and ICOS. In addition, the "antibody" in the "antibody capable of specifically binding to PD-1" or "anti-PD-1 antibody" means a full-length antibody, i.e., a full-length antibody composed of two heavy chains and two light chains linked through disulfide bond, preferably its monoclonal antibody.

The first arm is more preferably one that allows interaction between PD-1 and PD-L1, interaction between PD-1 and PD-L2, or both of these interactions. Whether the first arm allows interaction between PD-1 and PD-L1, interaction between PD-1 and PD-L2, or both of these interactions can be determined by measuring, for example, the effect of the first arm on binding of a soluble PD-1 protein to an immobilized PD-L1 protein or PD-L1 expressing cells using a publicly known method such as Biacore analysis, ELISA assay, flow cytometry, enzyme-linked immunosorbent assay (ELISA), fluorescence energy transfer assay (FRET) and Fluorometric Microvolume Assay Technology (FMAT (registered trademark)).

### Second arm specifically binding to CD19

In the present specification, the "second arm capable of specifically binding to CD19" means an antibody portion, at least including a VH and a VL of anti-CD 19 antibody, capable of specifically binding to CD 19 through associating with each other, regardless that it is included as a part of an antibody or antibody fragment thereof, or exists as not such a part but a single substance itself. Examples of the second arm include the Fab portion of an anti-CD19 antibody. Here, the "specifically binding to CD 19" is used as a feature that an antibody can directly bind to CD19 with the binding activity having at least an affinity (dissociation constant (Kd value)) higher than 1 × 10⁻⁵ M, more preferably 1 × 10⁻⁷ M, more preferably 1 × 10⁻⁹ M, but does not substantially bind to other proteins. In addition, the "antibody" in the "antibody capable of specifically binding to CD19" or "anti-CD 19 antibody" means a full-length antibody, i.e., a full-length antibody composed of two heavy chains and two light chains linked through disulfide bond, preferably its monoclonal antibody.

An isotype preferable as the PD-1/CD19 bispecific antibody of the present invention is an IgG antibody, more preferably an IgG₁ or IgG₄ antibody, still more preferably an IgG₁ antibody.

Among the bispecific antibodies of the present invention, when the antibody is an IgG₁ antibody, it is preferably a form in which mutation(s) substantially eliminating the binding to the Fc receptor has/have been introduced. Examples of the IgG₁ antibody include one in which leucine at position 234 according the EU numbering system is substituted with alanine, and/or leucine at position 235 is substituted with alanine on the two heavy chain constant regions or a hinge region, respectively. Alternatively, the IgG₁ antibody may also be one in which leucine at position 235 according to the EU numbering system is substituted with glycine and/or glycine at position 236 is substituted with arginine on the two heavy chain constant regions or a hinge region, respectively. Furthermore, it is more preferably one lacking the heavy chain C-terminal amino acid of these bispecific antibodies, for example, lysine at position 447 according to the EU numbering system. When the PD-1/CD19 bispecific antibody is an IgG₄ antibody, it is preferably an antibody in which serine at position 226 according to the EU numbering system, located in the hinge region is substituted with proline.

### Method for producing and purifying PD-1/CD19 bispecific antibody

The PD-1/CD19 bispecific antibody and antibody fragment thereof of the present invention can also be produced by the method disclosed in WO2014/051433, WO2013/157953 or WO2013/157954.

For example, they can be produced by performing gene transfer into a mammalian cell for transfection with an expression vector in which each of (1) a polynucleotide encoding a heavy chain having a VH of anti-PD-1 antibody, (2) a polynucleotide encoding a heavy chain having a VH of anti-CD 19 antibody, (3) a polynucleotide encoding a light chain having a VL of anti-PD-1 antibody and (4) a polynucleotide encoding a light chain having a VL of anti-CD 19 antibody is inserted, followed by expression and secretion of both of the heavy and light chains together.

Here, the host cell expressing the bispecific antibody of the present invention may be any host cell capable of performing gene transfer with an expression vector and expressing the transferred expression vector. Its referable examples include insect cells such as SF-9 and SF-21 cells, more preferably, mouse cells including CHO cells, BHK cells, SP2/0 cells and NS-0 myeloma cells, primate cells such as COS and Vero cells, and mammalian cells such as MDCK cells, BRL 3A cells, hybridomas, tumor cells, immortalized primary cells and embryonic retinal cells such as W138, HepG2, HeLa, HEK293, HT1080 or PER.C6. In selecting an expression system, mammalian cell expression vectors and hosts may be used to appropriately glycosylate an antibody. A human cell line, preferably PER.C6 is advantageously used to produce antibodies having glycosylation pattern corresponding to that in human.

Protein production in host cells transformed by gene transfer with an expression vector can be performed with reference to, e.g., Current Protocols in Protein Science (1995), Coligan JE, Dunn BM, Ploegh HL, Speicher DW, Wingfield PT, ISBN 0-471-11184-8, Bendig, 1988, and general guidelines, procedures and practical methods for maximizing the productivity in host cell culture can be performed with reference to Mammalian Cell Biotechnology: a Practical Approach (M. Butler, ed., IRL Press, 1991). Expression of antibodies in host cells is described in publications such as EP0120694, EP0314161, EP0481790, EP0523949, US4816567 and WO2000/63403.

Here, the condition for culturing the host cells can be optimized by a publicly known method, and the produced amount of protein can be optimized. The culture is performed by, for example, batch culture, fed-batch culture, continuous culture or hollow fiber culture in a dish, roller bottle or reaction vessel. In order to produce (continuous) recombinant protein by cell culture on a large scale, it is preferable to grow cells in a suspension. In addition, it is preferable to culture cells under conditions not including no animal or human-derived serum or animal or human-derived serum components.

Antibodies expressed on host cells and recovered from the cells or cell culture medium thereof by publicly known methods are purified using publicly known methods. Examples of the purification method include immunoprecipitation, centrifugation, filtration, size exclusion chromatography, affinity chromatography, cation and/or anion exchange chromatography and hydrophobic interaction chromatography. Furthermore, protein A or protein G affinity chromatography is preferably used (see, e.g., US4801687 and US5151504).

### [Pharmaceutical use]

The PD-1/CD19 bispecific antibody of the present invention is useful for prophylaxis, symptom progress-suppression and/or therapy of autoimmune disease or graft-versus-host disease (GVHD).

Examples of autoimmune disease for which the bispecific antibody of the present invention can be used as prophylactic, symptom progress-suppressive and/or therapeutic include Behcet disease, systemic lupus erythematosus, chronic discoid lupus erythematosus, multiple sclerosis (systemic scleroderma and progressive systemic scleroderma), scleroderma, polymyositis, dermatomyositis, periarteritis nodosa (polyarteritis nodosa and microscopic polyangiitis), aortitis syndrome (Takayasu arteritis), malignant rheumatoid arthritis, rheumatoid arthritis, juvenile idiopathic arthritis, spondylarthritis, mixed connective tissue disease, Sjogren syndrome, adult-onset Still's disease, vasculitis, allergic granulomatous angiitis, hypersensitivity angiitis, rheumatoid vasculitis, large-vessel vasculitis, ANCA-associated vasculitis (e.g., granulomatosis with polyangiitis and eosinophilic granulomatosis with polyangiitis), Cogan's syndrome, RS3PE syndrome, temporal arteritis, giant-cell arteritis, polymyalgia rheumatica, fibromyalgia syndrome, antiphospholipid antibody syndrome, eosinophilic fasciitis, IgG4-related diseases (e.g., primary sclerosing cholangitis and autoimmune pancreatitis), Guillain-Barre syndrome, myasthenia gravis, chronic atrophic gastritis, autoimmune hepatitis, non-alcoholic steatohepatitis, primary biliary cirrhosis, aortitis syndrome, Goodpasture's syndrome, rapidly progressive glomerulonephritis, anti-glomerular basement membrane nephritis, megaloblastic anemia, autoimmune hemolytic anemia, pernicious anemia, autoimmune neutropenia, idiopathic thrombocytopenic purpura, hyperthyroidism (Graves' disease (Basedow's disease)), Hashimoto's thyroiditis, autoimmune adrenal insufficiency, primary hypothyroidism, Addison's disease, idiopathic Addison's disease (chronic adrenal insufficiency), type I diabetes mellitus, slowly progressive type I diabetes mellitus, localized scleroderma, psoriasis, psoriatic arthritis, bullous pemphigoid, pemphigus, pemphigoid, herpes gestationis, linear IgA bullous skin disease, epidermolysis bullosa acquisita, alopecia areata, vitiligo, vitiligo vulgaris, Harada disease, autoimmune optic neuropathy, idiopathic azoospermia, recurrent fetal loss, inflammatory bowel diseases (e.g., ulcerative colitis and Crohn's disease), celiac disease, atopic dermatitis, neuromyelitis optica, chronic inflammatory demyelinating polyneuropathy, multifocal motor neuropathy, pulmonary alveolar proteinosis, autoimmune hemorrhagic disease XIII, relapsing polychondritis, sarcoidosis, ankylosing spondylitis, severe asthma, chronic urticaria, transplantation immunity, familial Mediterranean fever, eosinophilic sinusitis, dilated cardiomyopathy, food allergy, systemic mastocytosis, amyotrophic lateral sclerosis and inclusion body myositis.

In addition, the bispecific antibody of the present invention is particularly useful for prophylaxis, symptom progress-suppression and/or therapy of autoreactive B cell mediated diseases such as systemic lupus erythematosus, Basedow's disease, myasthenia gravis, autoimmune hemolytic anemia, autoimmune thrombocytopenia, asthma, cryoglobulinemia, primary bile duct sclerosis and/or pernicious anemia.

In the present invention, "therapy" means, for example, curing or improving a certain disease or its symptom, "prophylaxis" means preventing the onset of a certain disease or symptom or delaying those for a certain period of time, and "symptom progress-suppression" means suppressing the progression or worsening of symptom and stopping the progression of the disease state. The meaning of "prophylaxis" also includes recurrence-suppression.

The PD-1/CD19 bispecific antibody of the present invention is usually administered systemically or locally in a parenteral form. Specific examples of such administration methods include injection administration, transnasal administration, transpulmonary administration and transdermal administration. Examples of the injection administration include intravenous injection, intramuscular injection, intraperitoneal injection and subcutaneous injection. In the case of intravenous injection, administration by drip infusion is preferable. The dosage depends on age, weight, symptom, therapeutic effect, administration method, treating time, etc., but is usually administered parenterally once or several times a day, or continuously administered intravenously for 30 minutes to 24 hours a day, in a range of 0.1 µg/kg to 300 mg/kg, particularly preferably in a range of 0.1 mg/kg to 10 mg/kg in one administration for one adult. Of course, as mentioned above, since the dosage varies depending on various conditions, an amount smaller than the above dosage may be sufficient, or an amount beyond the above range may be necessary.

### [Pharmaceutical preparation]

When the PD-1/CD19 bispecific antibody of the present invention is formulated to be used as an injection or infusion solution for drip infusion, the injection or infusion solution may be in a form of an aqueous solution, suspension, or emulsion or may be formulated as a solid agent together with a pharmaceutically acceptable carrier such that the agent is dissolved, suspended, or emulsified in a solvent at the time of use. Usable examples of the solvent that is used in the injection or infusion solution for drip infusion include distilled water for injection, physiological saline, glucose solutions, isotonic solutions (e.g., solutions of sodium chloride, potassium chloride, glycerin, mannitol, sorbitol, boric acid, borax, propylene glycol, or the like) and the like.

Herein, examples of the pharmaceutically acceptable carrier include stabilizers, solubilizers, suspending agents, emulsifiers, soothing agents, buffering agents, preservatives, antiseptic agents, pH adjusters, antioxidants, and the like. Usable examples of the stabilizers include various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate, dibutylhydroxytoluene, and the like. Usable examples of the solubilizers include alcohols (e.g., ethanol and the like), polyalcohols (e.g., propylene glycol, polyethylene glycol and the like), nonionic surfactants (e.g., Polysorbate 20 (registered trademark), Polysorbate 80 (registered trademark), HCO-50 and the like) and the like. Usable examples of the suspending agents include glyceryl monostearate, aluminum monostearate, methyl cellulose, carboxymethyl cellulose, hydroxymethyl cellulose, sodium lauryl sulfate and the like. Usable examples of the emulsifiers include gum arabic, sodium alginate, tragacanth and the like. Usable examples of the soothing agents include benzyl alcohol, chlorobutanol, sorbitol and the like. Usable examples of the buffering agents include phosphate buffer, acetate buffer, borate buffer, carbonate buffer, citrate buffer, Tris buffer, glutamic acid buffer, epsilon aminocaproic acid buffer and the like. Usable examples of the preservatives include methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edeate, boric acid, borax and the like. Usable examples of the antiseptic agents include benzalkonium chloride, parahydroxybenzoic acid chlorobutanol and the like. Usable examples of the pH adjusters include hydrochloric acid, sodium hydroxide, phosphoric acid, acetic acid and the like. Usable examples of the antioxidants include (1) aqueous antioxidants such as ascorbic acid, cysteine hydrochloride, sodium bisulfate, sodium metabisulfite, sodium sulfite and the like, (2) oil-soluble antioxidants such as ascorbyl palmitate, butylated hydroxy anisole, butylated hydroxy toluene, lecithin, propyl gallate, α-tocopherol and the like, and (3) metal chelating agents such as citric acid, ethylenediaminetetraacetic acid, sorbitol, tartaric acid, phosphoric acid and the like.

The injection or infusion solution for drip infusion can be produced by performing sterilization or aseptic manipulation, for example, sterilization by filtration, with a filter in the final process and subsequently filling an aseptic container with it. The injection or infusion solution for drip infusion may be used by dissolving the vacuum dried or lyophilized aseptic powder (which may include a pharmaceutically acceptable carrier powder) in an appropriate solvent at the time of use.

### [Combination use or compounding agent]

The PD-1/CD19 bispecific antibody of the present invention may be used in combination with the other agent which is used for prophylaxis, symptom progress-suppression, recurrence-suppression and/or therapy of autoimmune disease or graft-versus-host disease (GVHD). In the present invention, when used in combination with the other agent (combination use), the dosage form may be a form of combination preparation in which both components are included in one formulation, or that of separate formulations. In such combination use, it is possible to complement the prophylactic, symptom progress-suppressive, recurrence-suppressive and/or therapeutic effect of the other agent or maintain or reduce the dosage or frequency of administration of the other agent. In a case of separately administering the bispecific antibody of the present invention and the other agent, both of them may be simultaneously administered for a certain period of time, and then only the bispecific antibody or only the other agent may be administered. Alternatively, the bispecific antibody of the present invention may be previously administered, and after completion of its administration, the other agent may be administered. Conversely, the other agent may be previously administered, and then the bispecific antibody of the present invention may be administered. The administration methods may be the same as or different from each other. The present invention also can provide a kit including a formulation containing the bispecific antibody of the present invention and a pharmaceutical preparation containing the other agent. Here, the dosage of the other agent can be appropriately selected based on the dose in clinical use. The other agent may be a combination of any two or more agents at an appropriate ratio. Examples of the other agent include those already known and also those newly discovered future.

For example, in the case of applying the bispecific antibody of the present invention to prophylaxis, symptom progress-suppression, recurrence-suppression and/or therapy of type I diabetes mellitus, it may be used in combination with, for example, an insulin preparation (e.g., human insulin, insulin glargine, insulin lispro, insulin detemir, insulin aspart or the like), sulfonylurea agent (e.g., glibenclamide, gliclazide, glimepiride or the like), quick-acting insulin secretion promoter (e.g., nateglinide or the like), biguanide preparation (e.g., metformin or the like), insulin sensitizer (e.g., pioglitazone or the like), α-glucosidase inhibitor (e.g., acarbose, voglibose or the like), diabetic neuropathy therapeutic agent (e.g., epalrestat, mexiletine, imidapril or the like), GLP-1 analog preparation (e.g., liraglutide, exenatide, lixisenatide or the like) or DPP-4 inhibitor (e.g., sitagliptin, vildagliptin, alogliptin or the like).

For example, in the case of applying the bispecific antibody of the present invention to prophylaxis, symptom progress-suppression, recurrence-suppression and/or therapy of multiple sclerosis, it may be used in combination with, for example, a steroid drug (e.g., cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, triamcinolone acetonide, dexamethasone, dexamethasone acetate, dexamethasone sodium phosphate, dexamethasone palmitate, paramethasone acetate, betamethasone or the like), interferon β-1a, interferon β-1b, glatiramer acetate, mitoxantrone, azathioprine, cyclophosphamide, cyclosporin, methotrexate, cladribine, adrenocorticotropic hormone (ACTH), corticotropin, mizoribine, tacrolimus, fingolimod, alemtuzumab and the like.

For example, in the case of applying the bispecific antibody of the present invention to prophylaxis, symptom progress-suppression, recurrence-suppression and/or therapy of systemic lupus erythematosus, it may be used in combination with a steroid drug (e.g., steroid drugs mentioned above) or immunosuppressive agent (e.g., cyclosporin, tacrolimus, fingolimod and the like).

For example, in the case of applying the bispecific antibody of the present invention to prophylaxis, symptom progress-suppression, recurrence-suppression and/or therapy of rheumatoid arthritis, it may be used in combination with, for example, a steroid drug (e.g., steroid drugs mentioned above), anti-rheumatic drug (e.g., methotrexate, sulfasalazine, bucillamine, leflunomide, mizoribine, tacrolimus or the like), anti-cytokine drug (e.g., infliximab, adalimumab, tocilizumab, etanercept, abatacept, golimumab certolizumab or the like) or the like

In the case of applying the bispecific antibody of the present invention to prophylaxis, symptom progress-suppression, recurrence-suppression and/or therapy of other autoimmune diseases or autoreactive B cell mediated diseases, it may be used in combination with any one or more of the above-mentioned other drugs.

The present invention will now be described in more detail by the following Examples, but the scope of the present invention is not limited thereto. Various changes and modifications can be made by those skilled in the art based on the description of the present invention, and such changes and modifications are also included in the present invention.

### EXAMPLES

### Example 1: Preparation of B cell-targeting PD-1 agonist

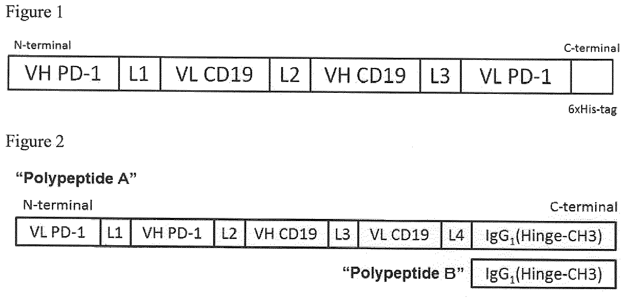

The "B cell-targeting PD-1 agonist" in this Example is a single-chain diabody (hereinafter, abbreviated as scDb) composed of the VH and VL of anti-human CD19 antibody and the VH and VL of anti-human PD-1 antibody. Figure 1 shows an outline of its structure. The scDb was obtained by inserting a DNA prepared by ligating DNAs encoding variable regions of each antibody and peptide linkers in a predetermined order into an expression vector, followed by expression in animal cells (Free Style 293F cells). The scDb produced in the culture supernatant was recovered and then purified by Protein A affinity chromatography and size exclusion chromatography. There are two types of scDb prepared in this Example, one being "B cell-targeting PD-1 agonist X" and the other being "B cell-targeting PD-1 agonist M". The anti-human CD19 antibody and anti-human PD-1 antibody composing the B cell-targeting PD-1 agonists X and M, respectively, were obtained in accordance with a method based on a publicly known antibody acquisition method. Furthermore, cDNAs encoding the VH and VL, respectively, were isolated by a publicly known method and used to construct a DNA expressing the scDb of this Example.

### Example 2: Preparation of B cell-targeting PD-1 agonist M-Fc

The "B cell-targeting PD-1 agonist M-Fc" in the Example is a heterodimer composed of two proteins, which are a polypeptide (hereinafter, referred to as polypeptide A) in which the human IgG₁ constant region from the hinge site to the CH3 region (hereinafter, abbreviated as "IgG₁ constant region (hinge/CH3)") is fused to bispecific sc(Fv)₂ composed of the VH and VL of anti-human CD19 antibody and the VH and VL of anti-human PD-1 antibody, and a polypeptide (hereinafter, referred to as polypeptide B) composed of only the IgG₁ constant region (hinge/CH3). The heterodimer has the mutation by which the Fcγ receptor-binding property is eliminated. Figure 2 shows an outline of its structure.

A DNA encoding the polypeptide A of the B cell-targeting PD-1 agonist M-Fc was produced by ligating DNAs respectively encoding the VH and VL of anti-human CD19 antibody and the VH and VL of anti-human PD-1 antibody obtained in Example 1 for producing the B cell-targeting PD-1 agonist M with DNAs encoding peptide linkers in a predetermined order. As for a DNA encoding the IgG₁ constant region (hinge/CH3) composing a part of the polypeptides A and B, its cDNA was obtained by a PCR method based on the publicly known gene information. Expression vectors into which each DNA sequence encoding the polypeptides A and B were inserted were subjected to gene transfer into the Free Style 293F cells for expression, followed by production in culture supernatant. The culture supernatant was collected and then purified by Protein A affinity chromatography and size exclusion chromatography for preparation.

### Example 3: Evaluation of binding property of B cell-targeting PD-1 agonist

Flow cytometry using human PD-1-expressing CHO-S cells was used to evaluate the binding property of the B cell-targeting PD-1 agonists to bind to PD-1 expressed on the human PD-1-expressing CHO-S cells. B cell-targeting PD-1 agonist X and M have 6×His tags added at their C terminal, so that an Alexa Fluor 488-labeled anti-His tag antibody (MBL, Model No. D291-A48) was used to detect each agonist bound to the same cells. On the other hand, B cell-targeting PD-1 agonist M-Fc contains the human IgG₁ constant region, so that the PE-labeled anti-human IgG-Fc antibody (Thermo Fisher Scientific, Model No. H10104) was used to detect B cell-targeting PD-1 agonist M-Fc bound to the same cells.

It was confirmed that both of B cell-targeting PD-1 agonists bind to human PD-1-expressing CHO-S cells. On the other hand, since the binding to CHO-S cells used as a control was not observed, it was confirmed that their bindings were specific to expressed PD-1.

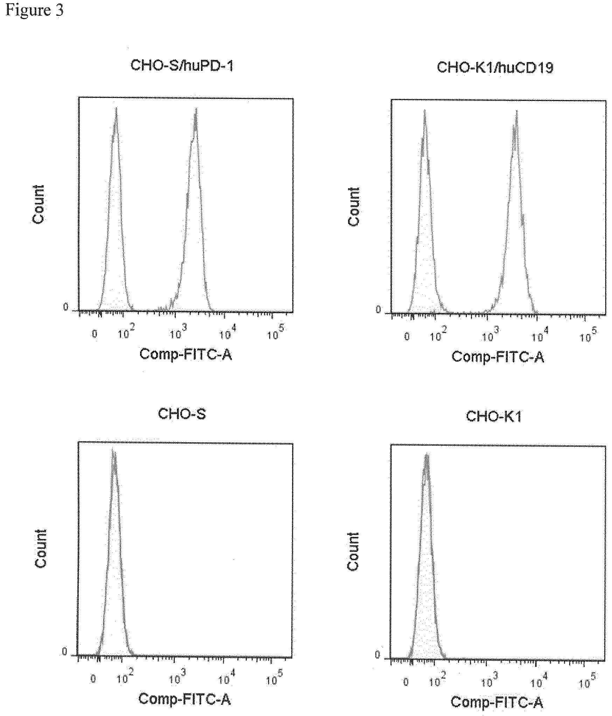

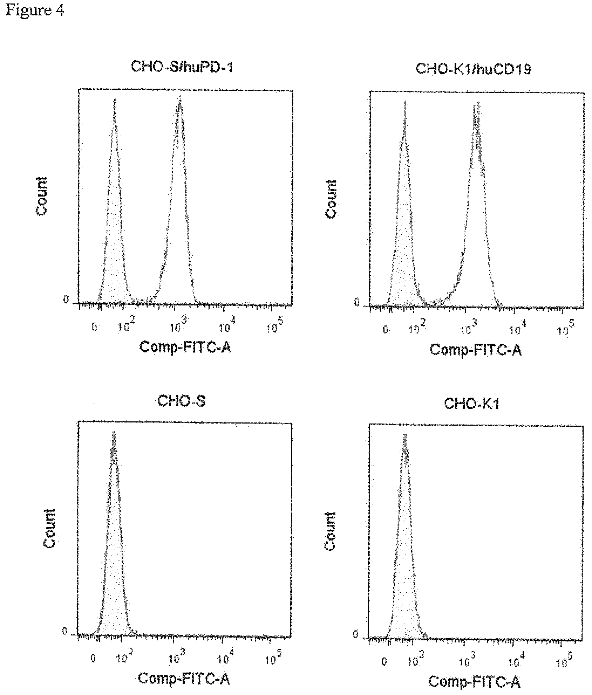

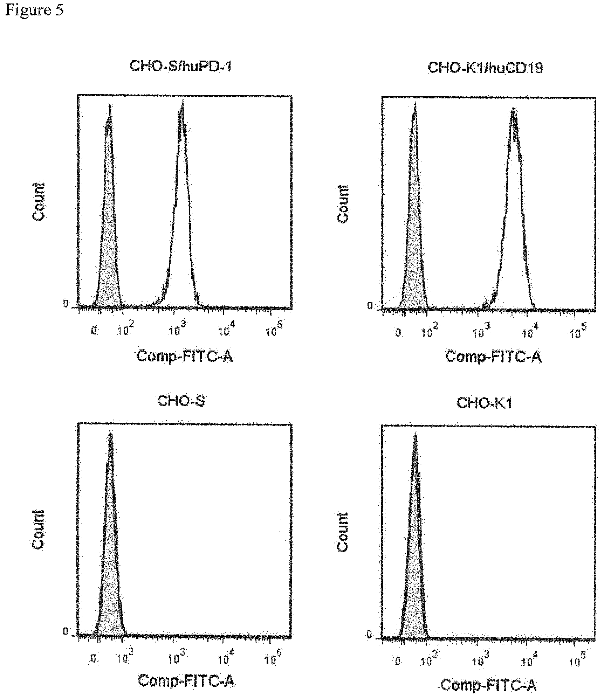

Subsequently, it was confirmed by the same method that the B cell-targeting PD-1 agonist specifically bound to human CD19. The above results are shown in Figures 3 to 5.

### Example 4: Effect of B cell-targeting PD-1 agonist on human B cells

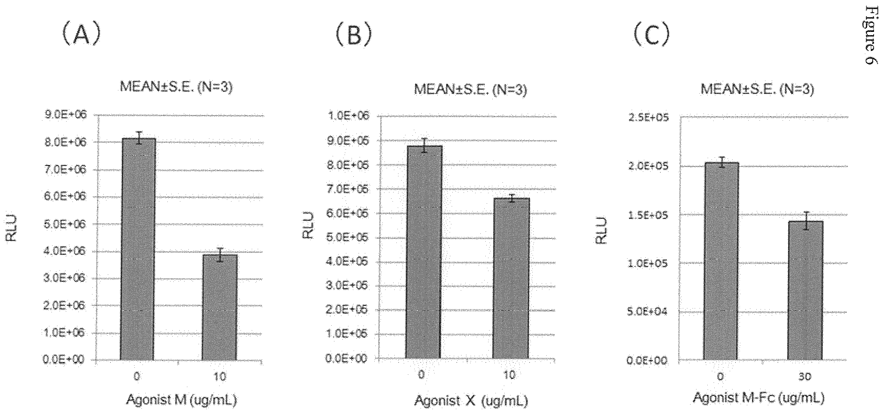

Human B cells were separated from normal human-derived peripheral blood mononuclear cells (LONZA, Model No. CC-2702) using B Cell Isolation Kit II human (Miltenyi Biotec, Model No. 130-091-151). The human B cells were seeded on cell culture plates, and the mouse anti-human CD79B antibody (LifeSpan Biosciences, Inc., Model No. LS-C134648), goat anti-mouse IgG-Fcy fragment F(ab')₂ antibody (Jackson ImmunoResearch, Model No. 115-006-008) and B cell-targeting PD-1 agonist X or M or M-Fc were added, followed by culture for 3 days. After 3 days of culture, the amount of ATP in the culture solution was measured using CellTiter-Glo2.0 Reagent (Promega, Model No. G924B). The result is shown in Figure 6. The B cell-targeting PD-1 agonist suppressed cell proliferation of the activated human B cells *in vitro.*

### Example 5: Effect of B cell-targeting PD-1 agonist M-Fc on human B cells

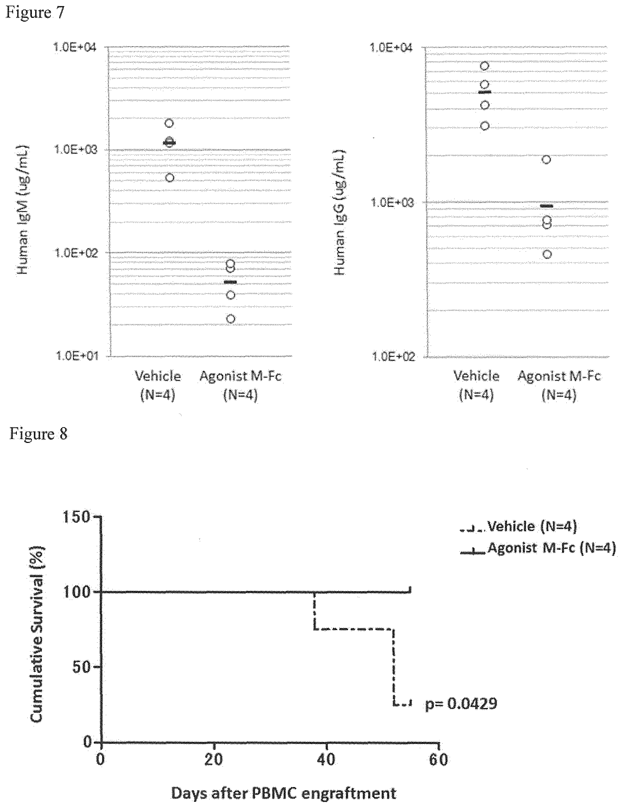

*In vivo* effect on human antibody production was evaluated using NOD.Cg-PrkdcscidI12rgtm1Wjl/SzJ mice (hereinafter, abbreviated as NSG mice) into which normal human-derived peripheral blood mononuclear cells (LONZA, Model No. CC-2702) were transplanted. For each NSG mouse, 1 × 10⁷ normal human-derived peripheral blood mononuclear cells were transplanted. On the day of transplantation and on days 7, 14, 17, 21, 24 and 28 thereof, 6 mg/kg of B cell-targeting PD-1 agonist M-Fc was intraperitoneally administered once a day. On day 28 of transplantation, blood was collected from tail vein to prepare serum. Human IgM contained in serum was quantified using ELISA kit (eBioscience, Model No. 88-50620). Human IgG contained in serum was quantified using ELISA kit (eBioscience, Model No. 88-50550). The result is shown in Figure 7. B cell-targeting PD-1 agonist M-Fc suppressed in vivo human IgM and IgG production, respectively.

### Example 6: Effect of B cell-targeting PD-1 agonist M-Fc in xenograft GVHD mouse model

Using NSG mice into which normal human-derived peripheral blood mononuclear cells (LONZA, Model No. CC-2702) were transplanted, the effect on xenograft GVHD was evaluated. For each NSG mouse, 1 × 10⁷ normal human-derived peripheral blood mononuclear cells were transplanted. On the day of transplantation and on days 7, 14, 17, 21, 24 and 28 thereof, 6 mg/kg of B cell-targeting PD-1 agonist M-Fc was intraperitoneally administered once a day. Survival curves up to day 55 of transplantation in the group to which the vehicle was administered and that to which B cell-targeting PD-1 agonist M-Fc was administered were generated by the Kaplan-Meier method, respectively, and compared between groups by the log rank test. The result is shown in Figure 8. B cell-targeting PD-1 agonist M-Fc exhibited effectiveness against xenograft GVHD.

### Example 7: Effect of B cell-targeting PD-1 agonist on human memory T cells

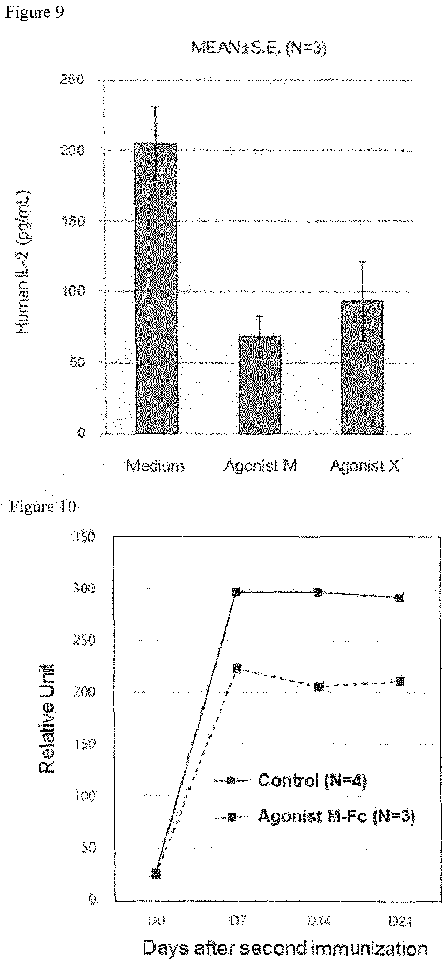

Normal human-derived peripheral blood mononuclear cells (LONZA, Model No. CC-2702) were seeded on a cell culture plate, and tetanus toxoid (List Biological Labs, Inc., Model No. 191A) and the B cell-targeting PD-1 agonist X or M were added, followed by culture for 4 days. IL-2 contained in the culture supernatant after 4 days of culture was quantified using the Bio-Plex Pro human cytokine panel (BIO-RAD, Model No. M50000007A). The result is shown in Figure 9. The B cell-targeting PD-1 agonist suppressed production of cytokine from human memory T cells *in vitro.*

### Example 8: Effect of B cell-targeting PD-1 agonist M-Fc on immune response to thymus-dependent antigen

Using human CD19/human PD-1 knock-in C57BL/6 mice in which extracellular regions of CD19 and PD-1 genes were substituted with human CD19 and PD-1 genes, respectively, *in vivo* effect on immune response to anti-KLH antibody production induced by immunization with Keyhole Limpet Hemocyanin (KLH), a thymus-dependent antigen, was evaluated. A suspension as an inducer was prepared by thoroughly mixing 0.2 mg/mL KLH (Thermo Fisher, Model No. 77600) with an equal amount of Alum (Thermo Fisher, Model No. 77161). 200 µL of the inducer was administered into abdominal cavity of the mice. From the day of immunization, blood was collected from tail vein daily, and serum was prepared. On the day of immunization and on days 7, 14, 22, 29 and 41 thereof, the antibody titers against KLH in serum were measured, and allocation was made to the control group and the group to which B cell-targeting PD-1 agonist M-Fc was administered such that the antibody titers at respective time points were equal. On day 41 of immunization, 200 µL of the inducer was administered again into abdominal cavity (secondary immunization). On days 3, 7, 10, 14 and 17 of secondary immunization, B cell-targeting PD-1 agonist M-Fc was intraperitoneally administered once a day at a dosage of 3 mg/kg. The antibody titers against KLH in serum on the day of secondary immunization and on days 7, 14 and 21 thereof were measured. The binding to KLH immobilized on ELISA plates was detected by HRP-labeled goat anti-mouse IgG antibodies (Southern Biotec, Model No. 1030-05) to calculate the antibody titer against KLH in serum by a relative quantification method based on a calibration curve generated using mouse anti-KLH monoclonal antibodies (BioLegend, Model No. 408402). The result is shown in Figure 10. B cell-targeting PD-1 agonist M-Fc suppressed immune response to the thymus-dependent antigen.

The human CD19/human PD-1 knock-in C57BL/6 mice can be prepared in accordance with the method described in Genesis. 2009 Jun; 47(6): 414-22.

### Example 9: Preparation of mouse B cell-targeting PD-1 agonists-Fc-1 and -2

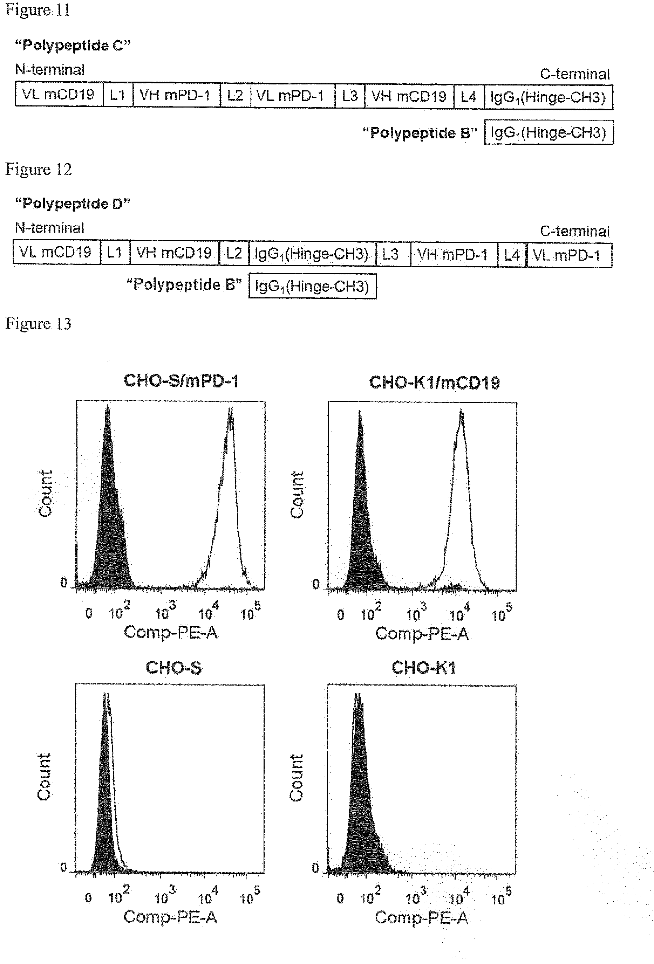

"Mouse B cell-targeting PD-1 agonist-Fc-1" in the Example is a heterodimer comprising two proteins, which are a polypeptide (hereinafter, referred to as polypeptide C) in which IgG₁ constant region (hinge/CH3) is fused to the scDb composed of the VH and VL of anti-mouse CD19 antibody and the VH and VL of anti-mouse PD-1 antibody, and polypeptide B. The heterodimer has the mutation by which the Fcγ receptor-binding property is eliminated. Figure 11 shows an outline of its structure.

On the other hand, "mouse B cell-targeting PD-1 agonist-Fc-2" in the Example is a heterodimer comprising two proteins, which are a polypeptide (hereinafter, referred to as polypeptide D) in which two kinds of scFvs composed of the VH and VL of anti-mouse CD19 antibody and the VH and VL of anti-mouse PD-1 antibody are fused to the N-terminal and C-terminal of IgG₁ constant region (hinge/CH3), respectively, and polypeptide B. The heterodimer also has the mutation by which the Fcγ receptor-binding property is eliminated. Figure 12 shows an outline of its structure.

DNAs respectively encoding the VH and VL of anti-mouse CD19 antibody and the VH and VL of anti-mouse PD-1 antibody were obtained in accordance with a method based on the publicly known base sequence analyzing method for a monoclonal antibody variable region using a total RNA prepared from an anti-mouse CD19 monoclonal antibody-producing hybridoma and anti-mouse PD-1 monoclonal antibody-producing hybridoma. For preparation, each obtained DNAs were ligated together with DNAs encoding peptide linkers in a predetermined order. As for a DNA encoding IgG₁ constant region (hinge/CH3), its cDNA was obtained by a PCR method based on the publicly known gene information. Expression vectors into which each DNA sequences encoding the polypeptide C and polypeptide B or polypeptide D and polypeptide B were inserted were subjected to gene transfer into the Free Style 293F cells for expression, followed by production in culture supernatant. The culture supernatant was collected and then purified by Protein A affinity chromatography and size exclusion chromatography for preparation.

Flow cytometry using mouse PD-1-expressing CHO-S cells was used to evaluate the binding property of the mouse B cell-targeting PD-1 agonist to bind to PD-1 expressed on the mouse PD-1-expressing CHO-S cells. The PE-labeled anti-human IgG-Fc antibody (Thermo Fisher Scientific, Model No. H10104) was used to detect mouse B cell-targeting PD-1 agonists-Fc-1 and -2 bound to the same cell.

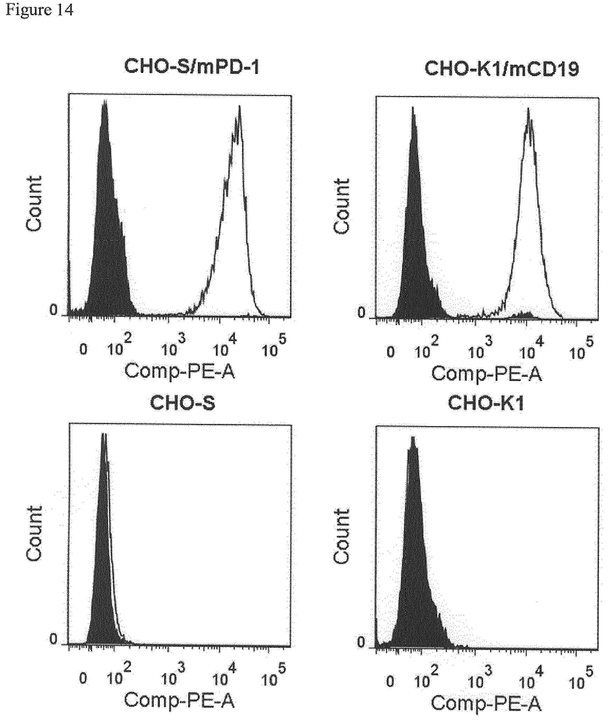

It was confirmed that both of mouse B cell-targeting PD-1 agonists bind to mouse PD-1-expressing CHO-S cells. On the other hand, since the binding to CHO-S cells used as a control was not observed, it was confirmed that its binding was specific to expressed PD-1. Subsequently, it was confirmed by the same method that the B cell-targeting PD-1 agonists specifically bind to mouse CD 19. The above results are shown in Figures 13 and 14.

### Example 10: In vivo effect of B cell-targeting PD-1 agonist on immune response in NP-OVA immunized mice

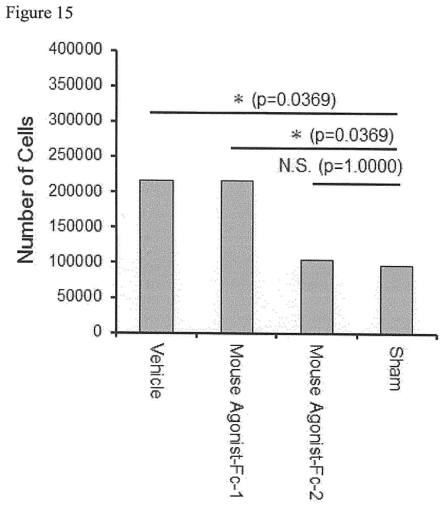

C57BL/6 mice were used to evaluate the *in vivo* effect on immune response to 4-hydroxy-3-nitrophenylacetyl ovalbumin (NP-OVA). A suspension as an inducer was prepared by thoroughly mixing 1 mg/mL NP-OVA (LGC Bioresearch, Model No. N-5051) with an equal amount of Alum (Thermo Fisher, Model No. 77161). 200 µL of the inducer was administered into abdominal cavity of the mice. On days 3, 7 and 10 of immunization, mouse B cell-targeting PD-1 agonist-Fc-1 and -2 were administered into abdominal cavity once a day at a dosage of 7 mg/kg. On day 14 of immunization, spleen was sampled and a single cell suspension was prepared using a cell strainer. The number of class switch memory B cells (B220⁺CD38⁺GL7⁻IgD⁻IgM⁻) was evaluated by flow cytometry using the single cell suspension after treatment with an ammonium chloride hemolyzing agent, and compared between groups using the Wilcoxon rank sum test. The result is shown in Figure 15.

In the group to which mouse B cell-targeting PD-1 agonist-Fc-2 was administered, the increase in class switch memory B cells was significantly suppressed. On the other hand, in the group to which mouse B cell-targeting PD-1 agonist-Fc-1 was administered, the increase in class switch memory B cells was not suppressed.

As antibodies against each surface antigen, a Brilliant Violet 605-labeled anti-mouse/human CD45R/B220 antibody (BioLgend, Model No. 103244), Brilliant Violet 711-labeled anti-mouse CD38 antibody (BD Bioscience, Model No. 740697), Alexa Fluor488 labeled antibody mouse/human GL7 antibody (BioLgend, Model No. 144612), APC-labeled anti-mouse IgD antibody (BioLegend, Model No. 405714) and PE/Dazzle594-labeled anti-mouse IgM antibody (BioLegend, Model No. 406530) were used.

### INDUSTRIAL APPLICABILITY

The PD-1/CD19 bispecific antibody or antibody fragment thereof of the present invention is useful for prophylaxis, symptom progress-suppression, recurrence-suppression and/or therapy for autoimmune disease or graft-versus-host disease (GVHD).

## Claims

1. A prophylactic, symptom progress-suppressive, recurrence-suppressive and/or therapeutic agent for autoimmune disease or graft-versus-host disease, containing a PD-1/CD19 bispecific antibody or antibody fragment thereof having the first arm capable of specifically binding to PD-1 and the second arm capable of specifically binding to CD19, as an active ingredient.

2. The agent according to claim 1, wherein the first arm contains a VH and VL of anti-PD-1 antibody.

3. The agent according to claim 1 or 2, wherein the second arm contains a VH and VL of anti-CD 19 antibody.

4. The agent according to any one of claims 1 to 3, wherein the PD-1/CD19 bispecific antibody has a structure in which the VH of anti-PD-1 antibody, the VL of anti-CD 19 antibody, the VH of anti-CD 19 antibody and the VL of anti-PD-1 antibody are linked via peptide linkers or directly to each other in this order from an N-terminal of the PD-1/CD19 bispecific antibody.

5. The agent according to any one of claims 1 to 3, wherein the PD-1/CD19 bispecific antibody has a structure in which the VL of anti-PD-1 antibody, the VH of anti-PD-1 antibody, the VH of anti-CD19 antibody and the VL of anti-CD19 antibody are linked via peptide linkers or directly to each other in this order from an N-terminal of the PD-1/CD19 bispecific antibody.

6. The agent according to claim 4 or 5, wherein the PD-1/CD19 bispecific antibody has a structure in which the N-terminal of a polypeptide having a human IgG constant region from a hinge site to a CH3 region is further linked via a peptide linker or directly to the C-terminal of the VL of anti-PD-1 antibody in the antibody of claim 4 or that of the VL of anti-CD19 antibody in the antibody of claim 5.

7. The agent according to claim 6, wherein the PD-1/CD19 bispecific antibody further has a structure containing the polypeptide having a human IgG constant region from a hinge site to a CH3 region.

8. The agent according to any one of claims 4 to 7, wherein the peptide linker is (Gly)×4-Ser or ((Gly)×4-Ser)×3.

9. The agent according to any one of claims 1 to 3, wherein the PD-1/CD19 bispecific antibody is in a form of diabody, bispecific sc(Fv)₂, bispecific minibody, bispecific F(ab')₂, bispecific hybrid antibody, covalent diabody, bispecific (FvCys)₂, bispecific F(ab'-zipper)₂, bispecific (Fv-zipper)₂, bispecific triple-chain antibody or bispecific mAb².

10. The agent according to any one of claims 1 to 3, wherein the PD-1/CD19 bispecific antibody is in a form of bispecific hybrid antibody.

11. The agent according to any one of claims 1 to 5 and 8 to 10, wherein the PD-1/CD19 bispecific antibody is an IgG antibody.

12. The agent according to claim 11, wherein the IgG antibody is an IgG₁ antibody or IgG₄ antibody.

13. The agent according to claim 6 or 7, wherein the isotype of the human IgG constant region from a hinge site to a CH3 region is IgG₁.

14. The agent according to any one of claims 1 to 13, wherein the PD-1 and the CD19 are human PD-1 and human CD19, respectively.

15. The agent according to any one of claims 1 to 14, wherein the PD-1/CD19 bispecific antibody is a humanized or fully human-type antibody.

16. The agent according to any one of claims 1 to 15, wherein the first arm of the PD-1/CD19 bispecific antibody allows interaction between PD-1 and PD-L1.

17. The agent according to any one of claims 1 to 16, wherein the PD-1/CD19 bispecific antibody binds to PD-1 and CD19 expressed on B cells.

18. A prophylactic, symptom progress-suppressive, recurrence-suppressive and/or therapeutic agent for autoimmune disease or graft-versus-host disease, having a humanized or fully human-type PD-1/CD19 bispecific antibody having the first arm capable of specifically binding to PD-1 and the second arm capable of specifically binding to CD19, as an active ingredient, wherein
(a) the first arm contains a VH and VL of anti-human PD-1 antibody, and the second arm contains a VH and VL of anti-human CD19 antibody,
(b) the PD-1/CD19 bispecific antibody binds to PD-1 and CD19 expressed on B cells,
(c) the first arm allows interaction between PD-1 and PD-L1, and
(d) the PD-1/CD19 bispecific antibody is in a form of bispecific hybrid antibody and/or IgG₁ antibody or IgG₄ antibody.

19. The agent according to any one of claims 1 to 18, wherein autoimmune disease is Behcet disease, systemic lupus erythematosus, chronic discoid lupus erythematosus, multiple sclerosis, scleroderma, polymyositis, dermatomyositis, periarteritis nodosa, aortitis syndrome, malignant rheumatoid arthritis, rheumatoid arthritis, juvenile idiopathic arthritis, spondylarthritis, mixed connective tissue disease, Sjogren syndrome, adult-onset Still's disease, vasculitis, allergic granulomatous angiitis, hypersensitivity angiitis, rheumatoid vasculitis, large-vessel vasculitis, ANCA-associated vasculitis, Cogan's syndrome, RS3PE syndrome, temporal arteritis, giant-cell arteritis, polymyalgia rheumatica, fibromyalgia syndrome, antiphospholipid antibody syndrome, eosinophilic fasciitis, IgG4-related diseases, Guillain-Barre syndrome, myasthenia gravis, chronic atrophic gastritis, autoimmune hepatitis, non-alcoholic steatohepatitis, primary biliary cirrhosis, aortitis syndrome, Goodpasture's syndrome, rapidly progressive glomerulonephritis, anti-glomerular basement membrane nephritis, megaloblastic anemia, autoimmune hemolytic anemia, pernicious anemia, autoimmune neutropenia, idiopathic thrombocytopenic purpura, hyperthyroidism, Hashimoto's thyroiditis, autoimmune adrenal insufficiency, primary hypothyroidism, Addison's disease, idiopathic Addison's disease, type I diabetes mellitus, slowly progressive type I diabetes mellitus, localized scleroderma, psoriasis, psoriatic arthritis, bullous pemphigoid, pemphigus, pemphigoid, herpes gestationis, linear IgA bullous skin disease, epidermolysis bullosa acquisita, alopecia areata, vitiligo, vitiligo vulgaris, Harada disease, autoimmune optic neuropathy, idiopathic azoospermia, recurrent fetal loss, inflammatory bowel diseases, celiac disease, atopic dermatitis, neuromyelitis optica, chronic inflammatory demyelinating polyneuropathy, multifocal motor neuropathy, pulmonary alveolar proteinosis, autoimmune hemorrhagic disease XIII, relapsing polychondritis, sarcoidosis, ankylosing spondylitis, severe asthma, chronic urticaria, transplantation immunity, familial Mediterranean fever, eosinophilic sinusitis, dilated cardiomyopathy, food allergy, systemic mastocytosis, amyotrophic lateral sclerosis or inclusion body myositis.

20. The agent according to any one of claims 1 to 19, wherein the agent is administered together with one or more agents selected from steroid drugs, interferon β-1a, interferon β-1b, glatiramer acetate, mitoxantrone, azathioprine, cyclophosphamide, cyclosporine, methotrexate, cladribine, adrenocorticotropic hormone (ACTH), corticotropin, mizoribine, tacrolimus, fingolimod, alemtuzumab, immunosuppressive agents, anti-rheumatic drugs and anti-cytokine drugs.

21. An isolated PD-1 /CD19 bispecific antibody or antibody fragment thereof having the first arm capable of specifically binding to PD-1 and the second arm capable of specifically binding to CD 19.

22. The antibody or antibody fragment thereof according to claim 21, wherein the first arm contains a VH and VL of anti-PD-1 antibody.

23. The antibody or antibody fragment thereof according to claim 21 or 22, wherein the second arm contains a VH and VL of anti-CD 19 antibody.

24. The antibody according to any one of claims 21 to 23, wherein the isolated PD-1/CD19 bispecific antibody has a structure in which the VH of anti-PD-1 antibody, the VL of anti-CD19 antibody, the VH of anti-CD 19 antibody and the VL of anti-PD-1 antibody are linked via peptide linkers or directly to each other in this order from the N-terminal of the isolated PD-1/CD19 bispecific antibody.

25. The antibody according to any one of claims 21 to 23, wherein the isolated PD-1/CD19 bispecific antibody has a structure in which the VL of anti-PD-1 antibody, the VH of anti-PD-1 antibody, the VH of anti-CD 19 antibody and the VL of anti-CD 19 antibody are linked via peptide linkers or directly to each other in this order from the N-terminal of the isolated PD-1/CD19 bispecific antibody.

26. The antibody according to claim 24 or 25, having a structure in which the N-terminal of a polypeptide having a human IgG constant region from a hinge site to a CH3 region further is linked via a peptide linker or directly to the C-terminal of the VL of anti-PD-1 antibody in the PD-1/CD19 bispecific antibody of claim 24 or that of the VL of anti-CD 19 antibody in the PD-1/CD19 bispecific antibody of claim 25.

27. The antibody according to claim 26, further having a structure including a polypeptide having a human IgG constant region from a hinge site to a CH3 region.

28. The antibody according to any one of claims 24 to 27, wherein the peptide linker is (Gly)×4-Ser or ((Gly)×4-Ser)×3.

29. The antibody or antibody fragment thereof according to any one of claims 21 to 23, being in a form of diabody, bispecific sc(Fv)₂, bispecific minibody, bispecific F(ab')₂, bispecific hybrid antibody, covalent diabody, bispecific (FvCys)₂, bispecific F(ab'-zipper)₂, bispecific (Fv-zipper)₂, bispecific triple-chain antibody or bispecific mAb².

30. The antibody or antibody fragment thereof according to any one of claims 21 to 23, being in a form of bispecific hybrid antibody.

31. The antibody or antibody fragment thereof according to any one of claims 21 to 25 and 28 to 30, being an IgG antibody.

32. The antibody or antibody fragment thereof according to claim 31, being an IgG₁ antibody or IgG₄ antibody.

33. The agent according to claim 26 or 27, wherein the isotype of the human IgG constant region from a hinge site to a CH3 region is IgG₁.

34. The antibody or antibody fragment thereof according to any one of claims 21 to 33, wherein PD-1 and CD19 are human PD-1 and human CD19, respectively.

35. The antibody or antibody fragment thereof according to any one of claims 21 to 34, being a humanized or fully human-type antibody.

36. The antibody or antibody fragment thereof according to any one of claims 21 to 35, wherein the first arm allows interaction between PD-1 and PD-L1.

37. The antibody or antibody fragment thereof according to any one of claims 21 to 36, binding to PD-1 and CD19 expressed on B cells.

38. A humanized or fully human-type isolated PD-1/CD 19 bispecific antibody having the first arm capable of specifically binding to PD-1 and the second arm capable of specifically binding to CD19,
(a) wherein the first arm contains a VH and VL of anti-human PD-1 antibody, and the second arm contains a VH and VL of anti-human CD19 antibody,
(b) which binds to PD-1 and CD19 expressed on B cells,
(c) wherein the first arm allows interaction between PD-1 and PD-L1, and
(d) which is in a form of bispecific hybrid antibody and/or IgG₁ or IgG₄ antibody.

39. A pharmaceutical composition, containing the isolated PD-1/CD19 bispecific antibody or antibody fragment thereof selected from any one of claims 21 to 37, or the humanized or fully human-type isolated PD-1/CD19 bispecific antibody of claim 38, and a pharmaceutically acceptable carrier.

40. A prophylactic, symptom progress-suppressive, recurrence-suppressive and/or therapeutic agent for autoreactive B cell mediated disease, containing the PD-1/CD19 bispecific antibody or antibody fragment thereof of any one of claims 21 to 37, or the humanized or fully human-type isolated PD-1/CD19 bispecific antibody of claim 38, as an active ingredient.

41. An autoreactive B cell suppressive agent, containing the PD-1/CD19 bispecific antibody or antibody fragment thereof of any one of claims 21 to 37, or the humanized or fully human-type isolated PD-1/CD19 bispecific antibody of claim 38, as an active ingredient.

42. A memory T cell activation suppressive agent, containing the PD-1/CD19 bispecific antibody or antibody fragment thereof of any one of claims 21 to 37, or the humanized or fully human-type isolated PD-1/CD19 bispecific antibody of claim 38, as an active ingredient.


SEQUENCE LISTING

<110>  ONO PHARMACEUTICAL CO., LTD.

<120>  PD-1/CD19 bispecific Antibody

<130>  P18-178WO-2

<150>  JP 2017-196039
<151>  2017-10-06

<160>  2

<170>  PatentIn version 3.5

<210>  1
<211>  5
<212>  PRT
<213>  Artificial Sequence

<400>  1

Gly Gly Gly Gly Ser
1               5


<210>  2
<211>  15
<212>  PRT
<213>  Artificial Sequence

<400>  2

Gly Gly Gly Gly Ser Gly Gly Gly Gly Ser Gly Gly Gly Gly Ser
1               5                   10                  15